# EUROPEAN PATENT SPECIFICATION

(11) **EP 0 746 255 B1**
(45) Date of publication and mention of the grant of the patent: **11.09.2002**
(21) Application number: 95902596.6
(22) Date of filing: 17.11.1994
(51) Int. Cl.: A61B 17/70

(54) **TRANSVERSE LINK FOR SPINAL IMPLANT SYSTEM**
TRANSVERSALE VERBINDUNG FÜR SPINALE IMPLANTATSYSTEME
LIAISON TRANSVERSALE POUR SYSTEME D'IMPLANT VERTEBRAL

(30) Priority: 19.11.1993 US 154986
(43) Date of publication of application: 11.12.1996
(73) Proprietor: CROSS MEDICAL PRODUCTS, INC., Columbus, OH 43228-1024 (US)
(72) Inventor: MELLINGER, Philip, A., Worthington, OH 43085 (US); BYRD, J., Abbott, III, Virginia Beach, VA 23452 (US); PUNO, Rolando, M., Prospect, KY 40059 (US)
(74) Representative: Stoner, Gerard Patrick
(86) International application number: US9413315
(87) International publication number: WO95013754

(56) References cited:
- EP-A- 0 558 121
- WO-A-91/16020
- FR-A- 2 645 427
- GB-A- 2 254 394
- US-A- 2 638 301
- US-A- 5 005 562
- US-A- 5 084 049
- US-A- 5 275 600
- US-A- 5 360 431

## Description

This invention relates to apparatus for immobilisation of the spine, and in particular to spinal stabilising arrangements in which elongate stabiliser rods are anchored to extend side-by-side along the spine and are linked by transverse linking members.

US-A-4805602 describes a rod-based system using anchor screws to hold the rods in place.

GB-A-2254394 describes a system of this kind in which hook elements are used to anchor the rods. Discrete transverse coupler assemblies are disclosed for connecting one rod to the other at a fixed spacing. The coupler has a C-shaped formation at each end to receive a respective one of the rods, with a turnbuckle sleeve in between to adjust their transverse spacing. The C-shaped recesses have openings which face perpendicularly to the axis of the turnbuckle. To keep the coupler in place on the rods, threaded tapered sleeves fit slidably on the rods to either side of each C-shaped hook and grip it from the sides, i.e. in the length direction of the rods.

FR-A-2645427 discloses simpler crosslinks in the form of a one-piece metal bar whose ends are bent around to form the C-shaped recesses for receiving the rods. The rods must be moved together to bring them between these oppositely-directed end hooks. Offset bevelled securing screws are screwed through from the back of the crosslink bar to hold the rods in place in the C-shaped recesses of the hooks. A variant in Fig. 4 of this document accommodates the possibility that the rods cannot be moved together in this way. In this variant, one end hook is directed inwardly along the bar as before; the other opens perpendicularly of the bar axis. However the securing screw for the latter is then at the end of the bar to keep the corresponding rod in place.

The present invention relates to new crosslinks for use with such spinal implant systems.

In a first aspect the present invention provides a linking member for linking transversely between first and second elongate stabiliser rods of a spinal stabilising arrangement when such rods are anchored to extend side-by-side along the spine, the linking member not comprising a vertebral anchor member for fixing into the vertebrae, but comprising
a first clamp, a second clamp and an intermediate link portion connecting between the first and second clamps, the direction in which the first and second clamps are spaced by said link portion being the length direction of the linking member;
each clamp having a retaining recess shaped to retain a respective said stabiliser rod, with an opening adapted to receive the stabiliser rod into the recess in a direction transverse to the longitudinal axis thereof, and
biasing means operable to secure each rod into the respective recess by engaging it at an offset from said longitudinal axis to bias it compressively into the recess;
characterised in that
said openings of the first and second clamp recesses face in the same direction along the length direction of the linking member, so that the stabiliser rods can be received into the respective recesses in the same direction.

In a second aspect, the invention provides a spinal stabilising arrangement comprising first and second elongate stabiliser rods, vertebral anchor members adapted to fix into the vertebrae to hold the stabiliser rods in position, and a linking member as defined above.

The recess can be a C-shaped well which receives the rod, a bevelled set screw being provided to bias the rod into position in the well.

One embodiment is adjustable in length, having the first and second clamp members telescopically linked together.

Another embodiment is a unitary structure, which may include a bendable neck for varying the distance between the clamps.

### BRIEF DESCRIPTION OF THE DRAWINGS

Fig. 1 is a side view of a spine showing an anchored rod in place;
Fig. 2 is a side view of a vertebral anchor showing its engagement with the rod;
Fig. 3 is a posterior view showing positioning of a joining link (not embodying the invention) between two rods secured side-by-side on the spine;
Fig. 4 is an exploded top view of an adjustable crosslink embodying the invention;
Fig. 5 is a side view of the crosslink;
Fig. 6 is a top view of the crosslink assembled;
Fig. 7 is a side view of the crosslink assembled;
Fig. 8 is a top view of a second embodiment, being a unified crosslink, in assembled condition, and
Fig. 9 is a side view of the unified crosslink of Fig. 8.

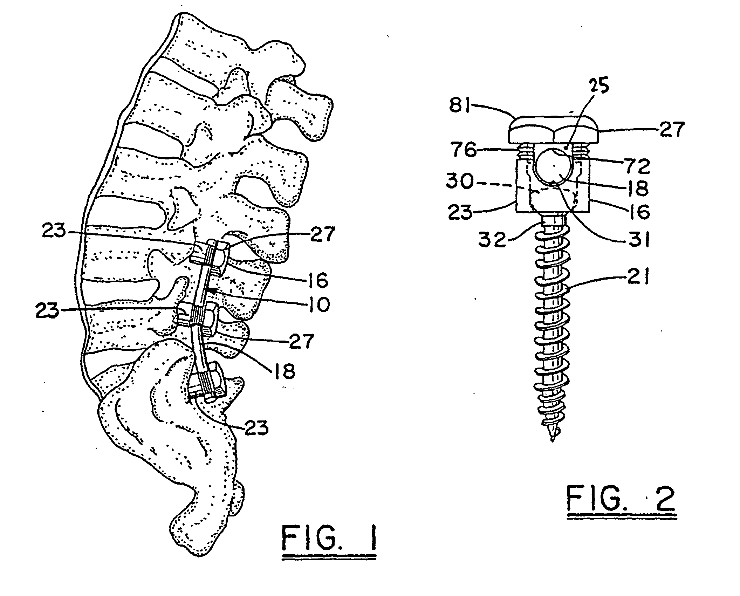

With reference to Figs 1 to 3, an anchor screw and rod system 10 includes two implant sets, to either side of the spinous processes. Each set comprises a plurality of vertebral anchors 16 and a rod 18 which is of sufficient length to span the length of spine to be immobilized.

Each anchor 16 is positioned on the dorsal side of the vertebra and in general, a separate anchor 16 is used for each vertebra comprised in the length of spine to be stabilized. The rod 18 is held by the anchors 16 posterior to the vertebra.

The rod 18 is generally made of 6.35mm (quarter inch) stainless-steel rod (316L), but could be made of any material which has suitable biocompatibility and material strength characteristics. The rod should be able to withstand lateral bending forces and torsion since the system may be used to correct spinal displacement and curvature. On the other hand, it is important that the rod 18 can be bent to a certain extent so that the rod can be bent to the proper curvature for the individual application.

The vertebral anchor 16 comprises a transpedicular screw 21, an anchor seat 23, a cap 25, and a nut 27. The various anchor parts 16 can be made of any suitably strong biocompatible material such as stainless steel. The screw 21 which is shown is a standard stainless steel cancellous screw with 6.5 mm thread diameter. It is available in various lengths. The anchor 16 was designed for use with this screw since the screw is readily available. It has a proven record in fracture fixation, and the size can be accommodated by the average adult pedicles of the lower thoracic, lumbar and the upper two sacral segments verte-brae.

The screw 21 includes a head 30 which accommodates a hex driver. The screw 21 includes a smooth shank 32 of 2-4 millimeters length which joins the rounded rear shoulder of the head 30. After insertion, the screw 21 extends from the curve formed on the dorsal side of the posterior neural arch.

The anchor seat 23 comprises a hollow cup portion which receives the screw and which includes opposing channels to receive the rod 18. The cup has a stepped central longitudinal opening having an upper inner diameter section of about 9,09mm (0.358") and a smaller lower diameter section which slightly exceeds the diameter of the head 30 of the screw 21. This step eliminates unwanted motion between the screw 21 and the anchor 23. This lower diameter section is about 8.20mm (0.323"). The screw 21 passes through the two sections of the opening within the rod support 23 until the rear shoulder of the screw 21 encounters a detaining flange within the central opening of the rod support 23. The flange has an internal surface at an angle of about 120 degrees and defines an opening which has a diameter that exceeds the diameter of the shank 32 but which is smaller than that of the head 30 of the screw 31. The diameter of the opening at the flange is about 6.9mm (0.27"). The internal surface of the detaining flange represents a sloped shoulder which forms a socket for the rear shoulder of the screw head 30. Thus, when the screw 21 engages the anchor seat 23, a limited ball-and-socket joint is formed which permits freedom of movement between the rod support 23 and the screw 21.

The anchor seat 23 has two opposing channels of the proper diameter to cradle the rod 18. The channels 51,52 form a rod-receiving cradle which is about 9.4mm (0.37") long.

The height of the anchor seat 23 generally determines the amount that the anchor 26 projects posterior of the vertebrae. This height ranges from 17 to 21mm (0.66 to 0.84"). However, if necessary, one or two washers may be added. These washers are smooth round washers having an outer diameter which corresponds to the diameter of the anchor seat, i.e. 13mm (0.5 inch) and a height of 1.6mm (0.063 inch). The washer fits around the screw 21 and is positioned under the seat between the bone and the seat 23. The washers are useful in indications where the patient is heavy or severely deformed.

on its external surface, the anchor seat 23 includes a threaded area 76. This area is 6.9mm (0.27 inch) deep to the thread runout. A 45 degree chamfer is included at the top to facilitate threading a nut 27 on the seat 23. The threads are at a count of 7.9 per cm (20 threads per inch). The nut 27 has a height of 4.8mm (0.19 inch) and includes a chamfered area 81 on its top surface. This chamfered area 81 blunts the edges of the nut and eliminates sharp edges which could otherwise irritate the soft tissues post-operatively. The seat 23 and a cap 25 complement each other to form a cylindrical unit onto which the nut 27 is threaded. On its bottom, the cap 25 includes an arch 72 transverse to the longitudinal axis of the cap 25.

The nut 27 includes internal threads which engage the external threaded area 76 on the anchor seat. The nut 27 is a hex nut which can be tightened relative to the cap 25.

As the nut 27 is rotated about the anchor seat 23, it cooperates with the top side of flanges on the cap to tighten the cap 25 in relation to the rod support 23. The rod 18 is grasped in the tunnel formed between the rod-receiving channel of the anchor seat 23 and the arch 72 of the cap 25.

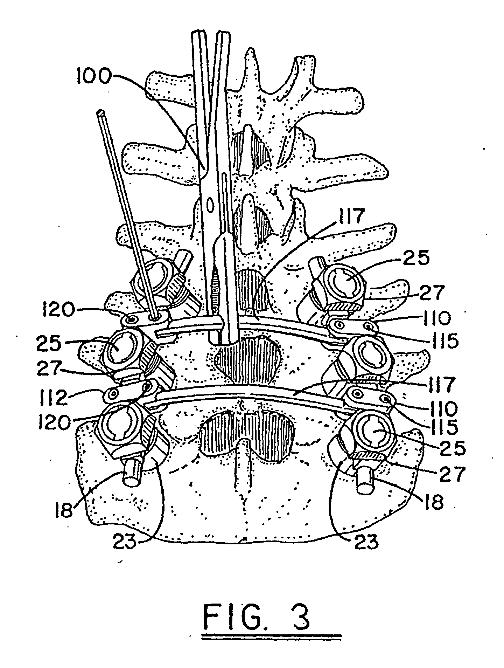

Fig. 3 shows how a crosslink 110 (not according to the invention) may be used to stabilize the rod members 18 against torsional rotation.

It is preferable that two crosslinks are used to form a rectangular construct. Each crosslink 110 comprises two clamps 112, each secured to the main rods 18. Specifically, each clamp 112 includes a rod receiving channel which accommodates the rod 18 and is locked into position relative thereto by a first set screw received in a bore. The clamp further includes a link opening which has a well to accommodate a link 117 axially transverse to the main rod 18. This link 117 may be, for example, a 4 mm Steinmann pin. The link 117 is locked into position by a second set screw which biases the link 117 into the well 119. The set screws 115 include a hexagonal opening 120 to receive a corresponding screwdriver. The screws 115 further include a terminal bevel at a 45 degree angle to facilitate locking the rod and link, respectively.

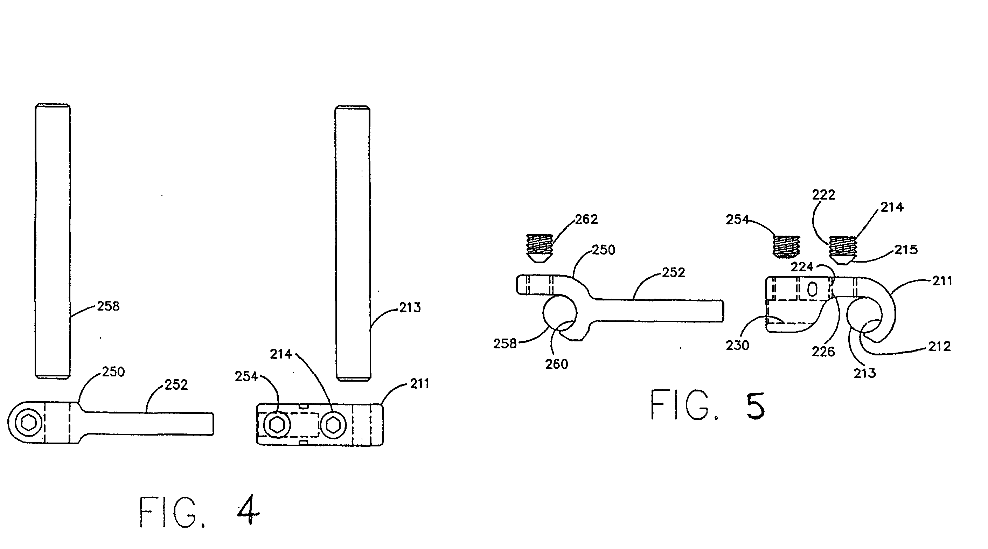

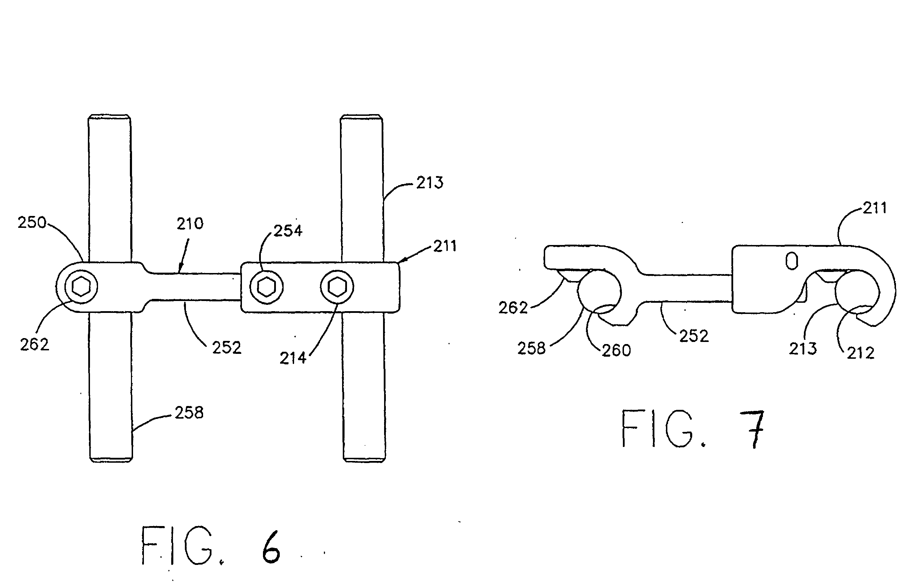

The invention relates to different crosslinks. A first embodiment of the invention crosslink assembly 210 is shown generally in Figs 4 to 7. The crosslink assembly has a first clamping member 211 which has a hooked well area 212 to receive a first spinal rod 213. The well area extends less than 360° and preferably from about 180° to about 270° so that the recess defined by the well has an opening which is large enough to easily accommodate the diameter of the stabilizer rod. Further, the terminus of the well has an angle which facilitates mounting the crosslink on the stabilizer rod.

A bevelled set screw 214 is offset with regard to the longitudinal axis of the rod 213. As the set screw 214 is tightened relative to the clamping member 211, the bevelled portion 215 biases the rod 213 into position against the well 212. The set screw 214 has external threads 222 which engage internal threads 224 in an internal bore 226 of the clamping means 211.

In addition, the clamping means 211 includes a second bore 230 having a central axis substantially perpendicular to the first bore 226. The second bore 230 receives a link/clamping means 250. The link/clamping means 250 specifically includes a link portion 252 having a longitudinal axis which is aligned with the longitudinal axis of the second bore 230. The link member 250 can be telescoped in and out of the second bore in order to adjust the distance that the crosslink spans between the two linked rods. A bevelled set screw 254 secures the link member 252 in position in the second bore 230. The bevelled terminus includes a self-locking, high friction surface. At its other end, the link/clamping member 250 includes a second clamping means 250 having a well 260 which receives the second substantial parallel rod 258 which is biased in position by means of the bevelled set screw 262 in a manner similar to the set screw 214 which has a contoured high friction terminus such as serrations.

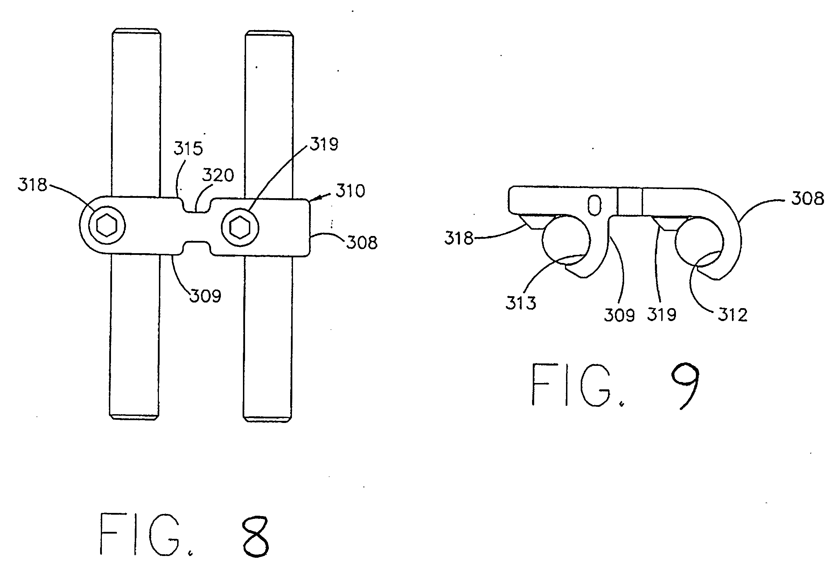

In a second embodiment of the invention shown in Figs 8 to 9, a crosslink 310 is provided having a substantially fixed length. When this variation is used, a variety of crosslinks are provided to accommodate different spans between the parallel rods. In this variation, the crosslink has first and second clamping members at either side of a unified elongate link member. The clamping members 308, 309 similarly consist of a C-shaped well portion 312, 313 which receives the rod, and a bevelled set screw 318, 319 which is offset relative to the longitudinal axis of the rod to bias the rod into contact in the well. The link portion 315 spanning the first clamping member 308 and the second clamping member 309 includes a necked area 320 which can be bent in order to accommodate variations in the relative location of the rods.

The unitary crosslink presents several advantages with regard to assembly of the crosslink with the stabilization rods. Naturally, it is advantageous to eliminate any assembly which might be required during spinal surgery. In addition, it is an advantage to provide a crosslink having hooked clamping means which are open in the same direction so that the crosslink can easily be assembled on the spinal rods by approaching the rods from the top and moving the crosslink in the direction of the openings in order to engage the rods simultaneously. Once the rods are engaged, the set screws may be tightened in order to lock the position of the crosslink relative to the rods. A variety of lengths are provided, ranging in overall length from 26.4 mm to 34.54 mm (from 1.040 inch to 1.360 inch) in order to accommodate the range of distances along the full length of the spine.

In use, the surgeon sequentially inserts an appropriate transpedicular screw 21 and anchor 23 seat assembly into each pedicle being instrumented. This is accomplished by using a hexagonal screwdriver. At the same time, a seat holder grips the seat, thereby preventing rotation when the screw 21 is finally tightened.

After all the screws and anchor seats are in place, an appropriate length of 6.35 mm rod is chosen and contoured with a French bender to fit the seats. The rod 18 is placed using a rod holder and secured on the seats with caps 25 which are placed over the rod using a rod holder and nuts 27 which are tightened down over the cap with the use of a T-wrench.

The procedure is repeated on the other side of the spine over the same number of vertebral levels.

Finally, Fig 3 shows how the crosslinks 110 (not embodying the invention) may be applied for added torsional stability. The crosslink is composed of two clamps 112, each of which is secured to one of the two main rods with set screws 115. The clamps are then bridged together by a 4 mm Steinmann pin which acts as a crosslink 117 which is cut to the length equivalent to the distance between the clamps. The Steinmann pin is secured to the clamp 117 with a second set screw. It is recommended that at least two sets of crosslinks are used to provide a more stable construct. The crosslinks in accordance with the first and second embodiments of the invention are used in a similar manner except that the adjustable link contemplates assembly adjustment and implantation while the unified piece is adjusted as need be by bending the linking portion.

## Claims

1. A linking member (210,310) for linking transversely between first and second elongate stabiliser rods (213,258) of a spinal stabilising arrangement when such rods are anchored to extend side-by-side along the spine, the linking member not comprising a vertebral anchor member for fixing into the vertebrae, but comprising
a first clamp (211,308), a second clamp (250,309) and an intermediate link portion (252, 315) connecting between the first and second clamps, the direction in which the first and second clamps are spaced by said link portion (252, 315) being the length direction of the linking member;
each clamp having a retaining recess (212,312;260,313) shaped to retain a respective said stabiliser rod, with an opening adapted to receive the stabiliser rod into the recess in a direction transverse to the longitudinal axis thereof, and
biasing means (214,262;318,319) operable to secure each rod into the respective recess by engaging it at an offset from said longitudinal axis to bias it compressively into the recess;
**characterised in that**
said openings of the first and second clamp recesses (212,312;260,313) face in the same direction along the length direction of the linking member, so that the stabiliser rods can be received into the respective recesses in the same direction.

2. A linking member according to claim 1 in which each biasing means is a set screw (214,262;318,319) received in a threaded bore (226) whose axis is offset from the longitudinal axis of the respective recess.

3. A linking member according to claim 2 in which the set screw has a beveled end surface (215) to contact the stabiliser rod for said biasing.

4. A linking member according to claim 3 in which the beveled end surface extends at a tangent to the stabiliser rod surface.

5. A linking member according to any one of claims 1 to 4 in which the link portion is formed in one piece with the first and second clamps (308,309) and includes a bendable neck (320) for varying the distance between the clamps.

6. A linking member according to any one of claims 1 to 4 in which the link portion has a link member (252) on the second clamp (250) received telescopically in a bore (230) on the first clamp (211), to provide an adjustable length between the first and second clamps.

7. A spinal stabilising arrangement comprising first and second elongate stabiliser rods (213,258);
vertebral anchor members (16) adapted to fix into the vertebrae to hold the stabiliser rods (213,258) in position, and
a linking member (210,310) according to any one of claims 1 to 6.

## Patentansprüche

1. Verbindungselement (210, 310) zur Querverbindung zwischen einem ersten und einen zweiten länglichen Stabilisatorstab (213, 258) einer Wirbelsäulen-Stabilisierungsanordnung, wenn solche Stäbe so verankert sind, dass sie nebeneinander die Wirbelsäule entlang verlaufen, wobei das Verbindungselement kein Wirbelverankerungselement zum Fixieren in den Wirbelknochen umfasst, sondern Folgendes umfasst:
eine erste Klammer (211, 308), eine zweite Klammer (250, 309) und einen Zwischenverbindungsabschnitt (252, 315) zur Verbindung zwischen der ersten und der zweiten Klammer, wobei die Richtung, in der die erste und die zweite Klammer durch den Verbindungsabschnitt (252, 315) beabstandet sind, die Längsrichtung des Verbindungselements ist;
wobei jede Klammer eine Rückhalteausnehmung (212, 312; 260, 313) aufweist, die so geformt ist, dass sie jeweils einen der Stabilisatorstäbe zurückhält, mit einer Öffnung, die so ausgebildet ist, dass sie den Stabilisatorstab in die Ausnehmung in einer Richtung aufnimmt, die zu seiner Längsachse quer verläuft, und
Vorspannmittel (214, 262; 318, 219), die betätigt werden können, um jeden Stab in der jeweiligen Ausnehmung zu sichern, indem sie in einer Versetzung von der Längsachse in Eingriff gebracht werden, um sie durch Zusammendrücken in die Ausnehmung vorzuspannen;
**dadurch gekennzeichnet, dass** die Öffnungen der ersten und der zweiten Klammerausnehmungen (212, 312; 260, 313) in Längsrichtung des Verbindungselements in die gleiche Richtung weisen, so dass die Stabilisatorstäbe in die jeweiligen Ausnehmungen in derselben Richtung aufgenommen werden können.

2. Verbindungselement nach Anspruch 1, bei dem jedes Vorspannmittel eine Stellschraube (214, 262; 318, 319) ist, die in einer Gewindebohrung (226) aufgenommen ist, deren Achse gegenüber der Längsachse der jeweiligen Ausnehmung versetzt ist.

3. Verbindungselement nach Anspruch 2, bei dem die Stellschraube eine abgeflachte Endfläche (215) zum Kontaktieren des Stabilisatorstabs zum Vorspannen umfasst.

4. Verbindungselement nach Anspruch 3, bei dem die abgeflachte Endfläche als Tangente zur Stabilisatorstabfläche verläuft.

5. Verbindungselement nach einem der Ansprüche 1 bis 4, bei dem der Verbindungsabschnitt mit der ersten und der zweiten Klammer (308, 309) einstückig ausgebildet ist und einen biegbaren Hals (320) umfasst, um den Abstand zwischen den Klammern zu variieren.

6. Verbindungselement nach einem der Ansprüche 1 bis 4, bei dem der Verbindungsabschnitt ein Verbindungselement (252) auf der zweiten Klammer (250) aufweist, das teleskopisch in einer Bohrung (230) auf der ersten Klammer (211) aufgenommen ist, um für eine einstellbare Länge zwischen der ersten und der zweiten Klammer zu sorgen.

7. Wirbelsäulen-Stabilisierungsanordnung, die einen ersten und einen zweiten länglichen Stabilisatorstab (213, 258);
Wirbelknochen-Verankerungselemente (16), die zum Fixieren in den Wirbelknochen ausgebildet sind, um die Stabilisatorstäbe (213, 258) in Position zu halten, sowie
ein Verbindungselement (210, 310) nach einem der Ansprüche 1 bis 6 umfasst.

## Revendications

1. Elément de liaison (210, 310) pour établir une liaison transversalement entre des première et seconde tiges de stabilisation oblongues (213, 258) d'un agencement de stabilisation vertébral lorsque de telles tiges sont ancrées pour s'étendre côte-à-côte le long du rachis, l'élément de liaison ne comprenant pas d'élément d'ancrage vertébral pour la fixation dans les vertèbres mais comprenant :
un premier organe de serrage (211, 308), un deuxième organe de serrage (250, 309) et une portion de liaison intermédiaire (252, 315) établissant une connexion entre les premier et second organes de serrage, la direction dans laquelle les premier et second organes de serrage sont espacés par ladite portion de liaison (252, 315) étant la direction en longueur de l'élément de liaison ;
chaque organe de serrage présentant un évidement de retenue (212, 312 ; 260, 313) configuré pour retenir une tige de stabilisation respective précitée, une ouverture étant conçue pour recevoir la tige de stabilisation dans l'évidement dans une direction transversale à son axe longitudinal et
des moyens de sollicitation (214, 262 ; 318, 319) fonctionnant pour fixer chaque tige dans l'évidement respectif en les mettant en prise avec celle-ci à un décalage dudit axe longitudinal pour la solliciter par compression dans l'évidement ;
**caractérisé en ce que** lesdites ouvertures des évidements (212, 312 ; 260, 313) des premier et second organes de serrage sont orientées dans la même direction le long de la direction en longueur de l'élément de liaison de telle sorte que les tiges de stabilisation peuvent être reçues dans les évidements respectifs dans la même direction.

2. Elément de liaison selon la revendication 1, où chaque moyen de sollicitation est une vis sans tête (214, 262 ; 318, 319) reçue dans un perçage taraudé (226) dont l'axe est décalé de l'axe longitudinal de l'évidement respectif.

3. Elément de liaison selon la revendication 2, dans lequel la vis sans tête présente une surface d'extrémité biseautée (215) pour venir en contact avec la tige de stabilisation en vue de ladite sollicitation.

4. Elément de liaison selon la revendication 3, où la surface d'extrémité biseautée s'étend selon une tangente à la surface de la tige de stabilisation.

5. Elément de liaison selon l'une des revendications 1 à 4, où la portion de liaison est réalisée en une pièce avec les premier et second organes de serrage (308, 309) et comporte un col (320) qui est apte à se courber pour faire varier la distance entre les organes de serrage.

6. Elément de liaison selon l'une des revendications 1 à 4, où la portion de liaison comporte un élément de liaison (252) sur le second organe de serrage (250) reçu télescopiquement dans un perçage (230) sur le premier organe de serrage (211) pour réaliser une longueur asjustable entre les premier et second organes de serrage.

7. Agencement de stabilisation vertébral comprenant des première et seconde tiges de stabilisation oblongues (213, 258) ;
des éléments d'ancrage vertébraux (16) aptes à être fixés dans les vertèbres pour maintenir les tiges de stabilisation (213, 258) en position et
un élément de liaison (210, 310) selon l'une des revendications 1 à 6.
